# EUROPEAN PATENT APPLICATION

(11) **EP 3 954 313 A1**
(43) Date of publication of application: **16.02.2022**
(21) Application number: 21165807.5
(22) Date of filing: 30.03.2021
(51) Int. Cl.: A61B 18/14, A61B 18/00

(54) **BALLOON PROBE FOR IRREVERSIBLE ELECTROPORATION**

(30) Priority: 13.08.2020 US 202016992224
(71) Applicant: Biosense Webster (Israel) Ltd, Yokneam, 2066717 (IL)
(72) Inventor: ADAWI, Eid, 2066717 Yokneam (IL); ZIV-ARI, Morris, 2066717 Yokneam (IL); AUERBACH, Shmuel, 2066717 Yokneam (IL)
(74) Representative: Carpmaels & Ransford LLP

(57) **Abstract**

An apparatus includes a shaft, configured for insertion into a body of a subject, an inflatable balloon coupled to a distal end of the shaft, and a plurality of electrodes coupled to a distal portion of the balloon that is perpendicular to a longitudinal axis of the distal end of the shaft when the balloon is inflated. Other embodiments are also described.

## Description

### FIELD OF THE INVENTION

The present invention relates to medical devices for the application of electrical energy to tissue.

### BACKGROUND

A conventional balloon probe (or "balloon catheter") typically comprises a spherical or ellipsoidal balloon, with multiple electrodes arranged along splines running across the surface of the balloon.

US Patent No. 5,846,196 describes an intraventricular multielectrode cardiac mapping probe having an electrode array assembly that is initially stored within the distal lumen of a delivery catheter. After placement of the tip of the catheter at a target location within the heart, the electrode array may be moved out of its retracted position to expand into a generally planar configuration to position electrodes for mapping of the heart chamber.

US Patent No. 9,060,756 describes a method of ablating body tissue, including (a) locating an inflatable balloon portion of a cryotherapy balloon catheter at a treatment site internal to a patient's body, and inflating the inflatable balloon portion, (b) employing electrodes that are disposed on an expandable surface of the inflatable balloon portion to electrically characterize body tissue at the treatment site, (c) ablating the body tissue by supplying a cryotherapy agent to the inflatable balloon portion to cool the body tissue to a therapeutic temperature, (d) employing the electrodes to determine whether the ablating caused desired electrical changes in the body tissue, and (e) repeating (c) and (d) when it is determined that the ablating did not cause the desired electrical changes.

### SUMMARY OF THE INVENTION

There is provided, in accordance with some embodiments of the present invention, an apparatus including a shaft, configured for insertion into a body of a subject, an inflatable balloon coupled to a distal end of the shaft, and a plurality of electrodes coupled to a distal portion of the balloon that is perpendicular to a longitudinal axis of the distal end of the shaft when the balloon is inflated.

In some embodiments, the distal portion of the balloon is perpendicular to the longitudinal axis of the distal end of the shaft by virtue of an angle between (i) the longitudinal axis of the distal end of the shaft and (ii) a normal line to the distal portion of the balloon at any point on the distal portion of the balloon being less than 30 degrees.

In some embodiments, the electrodes are arranged in a grid.

In some embodiments, the shaft extends through the balloon and is coupled to the distal portion of the balloon.

In some embodiments, the apparatus further includes at least one support rod proximally coupled to the shaft and distally coupled to the distal portion of the balloon.

In some embodiments, an area of the distal portion of the balloon is at least 10 mm².

In some embodiments, the balloon includes a distal surface including the distal portion of the balloon.

In some embodiments, the distal surface has a circular perimeter.

In some embodiments, the apparatus further includes one or more sensors coupled to the distal surface.

There is further provided, in accordance with some embodiments of the present invention, a method including inserting a shaft into a body of a subject. The method further includes, subsequently to inserting the shaft, inflating a balloon coupled to a distal end of the shaft. The method further includes, subsequently to inflating the balloon, using a plurality of electrodes coupled to a distal portion of the balloon that is perpendicular to a longitudinal axis of the distal end of the shaft, applying electrical energy to intrabody tissue of the subject.

In some embodiments, the tissue includes cardiac tissue.

In some embodiments, applying the electrical energy includes applying the electrical energy so as to cause irreversible electroporation (IRE) of the tissue.

In some embodiments, applying the electrical energy includes applying the electrical energy while all the electrodes coupled to the distal portion of the balloon contact the tissue.

### BRIEF DESCRIPTION OF THE DRAWINGS

The present invention will be more fully understood from the following detailed description of embodiments thereof, taken together with the drawings, in which:
Fig. 1 is a schematic illustration of a system for treating intrabody tissue of a subject, in accordance with some exemplary embodiments of the present invention;
Fig. 2 is a schematic illustration of an intrabody probe, in accordance with some exemplary embodiments of the present invention; and
Fig. 3 is a flow diagram for a method of using an intrabody probe, in accordance with some exemplary embodiments of the present invention.

### DETAILED DESCRIPTION OF EMBODIMENTS

### OVERVIEW

Some procedures, particularly irreversible electroporation (IRE) procedures, require that multiple electrodes contact the tissue simultaneously. However, this requirement may be difficult to satisfy using conventional probes, such as conventional balloon probes.

To address this challenge, embodiments of the present invention provide a balloon comprising, when inflated, a distal portion that is perpendicular to the longitudinal axis of the distal end of the probe shaft to which the balloon is coupled. A plurality of electrodes are coupled to the distal portion of the balloon. To apply electrical energy to tissue, e.g., during an IRE procedure, the electrodes are pressed against the tissue, and electric currents are then passed between the electrodes.

By virtue of the distal portion of the balloon being perpendicular to the longitudinal axis (without possessing significant curvature that would render parts of the distal portion non-perpendicular), most or all of the electrodes coupled to the distal portion may contact the tissue simultaneously. Moreover, the electrodes may be pressed against the tissue with a relatively large force, by applying an axial force to the probe shaft.

### SYSTEM DESCRIPTION

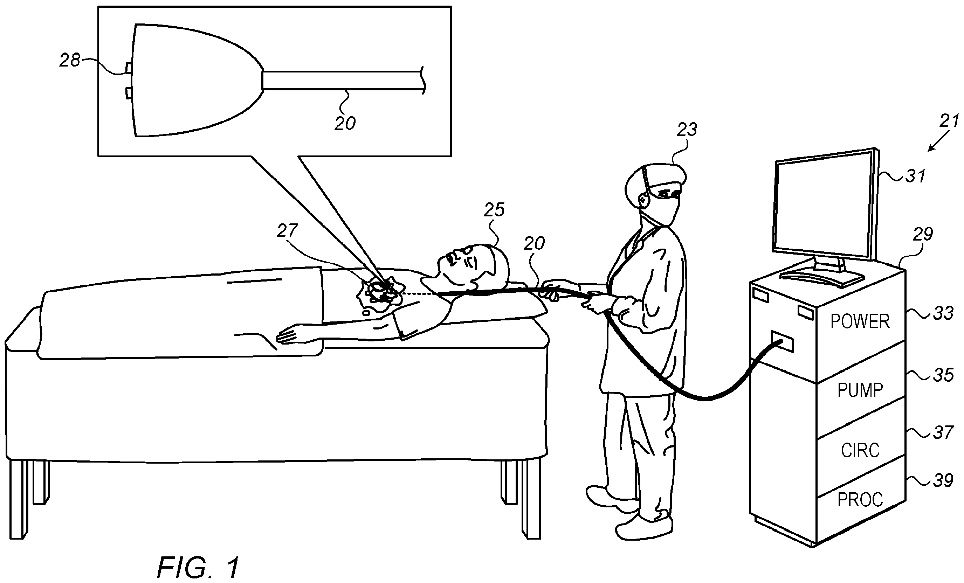

Reference is initially made to Fig. 1, which is a schematic illustration of a system 21 for treating intrabody tissue of a subject 25, in accordance with some embodiments of the present invention.

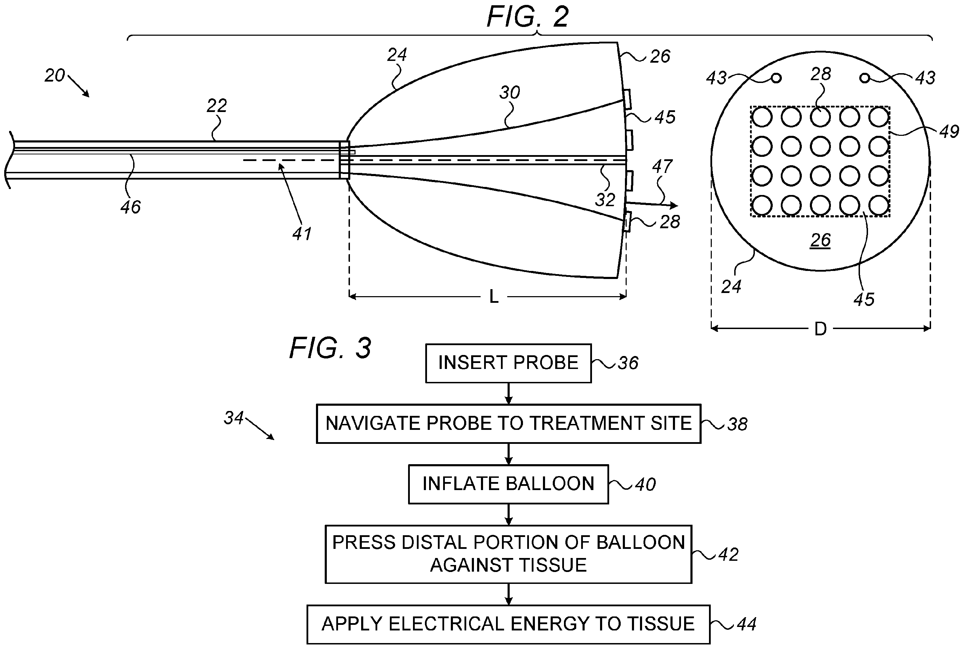

System 21 comprises an intrabody probe 20 comprising a plurality of electrodes 28 at its distal end. System 21 further comprises a power source 33 configured to supply electric signals to electrodes 28. Using system 21, a physician 23 may treat intrabody tissue of subject 25, such as tissue of the heart 27 of the subject, with electrical energy resulting from the electric signals passing between the electrodes. For example, as further described below with reference to Fig. 3, system 21 may be used to ablate or to cause IRE of the tissue.

Typically, probe 20 is proximally connected to a console 29, which contains the power source 33. As further described below with reference to Fig. 2, wires, which run through probe 20 from console 29 to electrodes 28, deliver electrical signals from the power source to the electrodes. In some embodiments, console 29 further contains a pump 35 configured to supply fluid to the distal end of the probe, as further described below with reference to Fig. 2. In some embodiments, the console 29 further contains a processor (PROC) 39 for controlling the power source 33, the pump 35, and/or other components of the system. The console 29 may further contain any other circuitry (CIRC) 37, such as circuitry for interfacing between processor 39 and any analog components of the system 21.

In some embodiments, system 21 further comprises a monitor 31. While physician 23 navigates the probe to the treatment site, and/or while electrical energy is applied to the tissue, processor 39 may cause monitor 31 to display relevant data. Such data may include, for example, an image of the distal end of the probe and/or of the treatment site, one or more properties (e.g., the temperature) of the tissue, and/or current treatment parameters, such as an amount of power supplied by power source 33 or an amount of pressure applied to the tissue by the probe.

Reference is now made to Fig. 2, which is a schematic illustration of intrabody probe 20, in accordance with some embodiments of the present invention. Fig. 2 shows both a longitudinal cross-section through probe 20 (left) and a view of the distal surface of the probe (right).

Probe 20 comprises a shaft 22 configured for insertion into the body of subject 25 (Fig. 1) . The probe further comprises an inflatable balloon 24 coupled to the distal end of shaft 22. Typically, the balloon is compliant, so as to conform to the intrabody tissue of the subject.

Balloon 24, when inflated, comprises a distal portion 45 that is perpendicular to the longitudinal axis 41 of the distal end of the shaft. (Hence, distal portion 45 is a distally-facing portion of the balloon.) A plurality of (e.g., at least two, three, or four) electrodes 28 are coupled to distal portion 45. (Distal portion 45 may thus be identified as the portion of the balloon contained within a minimum bounding curve 49 that encloses the electrodes.)

In some embodiments, distal portion 45 is slightly concave or convex, e.g., so as to conform to curvature in the wall of heart 27 (Fig. 1). Nonetheless, distal portion 45 may be described as being perpendicular to longitudinal axis 41, given that in the context of the present application, including the claims, the term "perpendicular" is broad enough so as to include slight deviations from strict perpendicularity. For example, the distal portion of the balloon may be perpendicular to longitudinal axis 41 by virtue of the angle between (i) longitudinal axis 41 and (ii) a normal line 47 to the distal portion of the balloon at any point on the distal portion of the balloon - i.e., at any point between any two of the electrodes - being less than 30 degrees, such as less than 20 or 10 degrees.

Typically, the balloon comprises a distal surface 26 comprising distal portion 45. Portions of distal surface 26 outside of distal portion 45 are not necessarily perpendicular to longitudinal axis 41. In some embodiments, the distal surface has a circular perimeter.

In some embodiments, one or more sensors 43, such as a temperature sensor and/or a pressure sensor, are coupled to surface 26, within or outside of distal portion 45. Output from such sensors may be carried over suitable wires to console 29 (Fig. 1) for processing by processor 39.

In general, the portion of balloon 24 that is proximal to surface 26 may have any suitable shape, such as a paraboloidal or conical shape. In some embodiments, additional electrodes are coupled to this portion of the balloon. Typically, the length L of the balloon is an increasing function of the size of surface 26; for example, L may be at least as large as D/2, D being the diameter of the surface.

In some embodiments, electrodes 28 are arranged in a grid. For example, four electrodes may be arranged in a 2 x 2 grid, or, as shown in Fig. 2, 20 electrodes may be arranged in a 5 x 4 grid.

In some embodiments, electrodes 28 are glued to the balloon, using a polyurethane adhesive or any other suitable adhesive. (In such embodiments, the electrodes may comprise respective printed circuit boards (PCBs).) In other embodiments, the electrodes are swaged onto the balloon, or are coupled to the balloon using any other suitable technique.

Respective wires 30, which pass through shaft 22 and through the balloon, are distally connected to electrodes 28. (For ease of illustration, only two wires 30 are shown in Fig. 2.) The wires are configured to connect the electrodes to power source 33 (Fig. 1) .

In general, the preferred size of each electrode is a function of the thickness of the tissue that is to be treated. For example, for treatment of an atrial wall, the surface area of each of the electrodes may be between 3 and 30 mm²; for example, each of the electrodes may be circular with a diameter between 2 and 6 mm. (It is noted that alternatively to a circular shape, the electrodes may have any other suitable shape, such as an elliptical, rectangular, or toroidal shape.) For treatment of a (thicker) ventricular wall, the surface area of each of the electrodes may be greater than 30 mm².

Typically, the area of distal portion 45 is at least 10 mm², such that distal portion 45 is large enough to accommodate at least two circular electrodes, each having a diameter of 2 mm and spaced apart from one another by 1 mm.

In some embodiments, shaft 22 extends through the balloon and is (mechanically) coupled to distal portion 45. In other embodiments, at least one support rod 32 is proximally coupled to the shaft and distally coupled to distal portion 45. Shaft 22 or rod 32 may thus stabilize the distal portion of the balloon as the distal portion of the balloon is pressed against the tissue.

To inflate the balloon, a fluid, such as saline, may be pumped by pump 35 (Fig. 1), or by another pump that is separate from console 29, through the shaft and into the balloon. Alternatively, the fluid may be passed from a syringe through the shaft and into the balloon. Typically, the fluid flows through one or more inflation tubes 46 running through the shaft.

In some embodiments, distal portion 45, another portion of surface 26, and/or a more proximal portion of the balloon is shaped to define one or more apertures. In such embodiments, a fluid, such as saline, may be passed through the apertures, e.g., during an ablation procedure, so as to facilitate the application of electrical energy. This fluid may be the same fluid used to inflate the balloon, i.e., fluid may be continuously passed through the balloon so as to keep the balloon inflated and also to facilitate the application of electrical energy.

Reference is now made to Fig. 3, which is a flow diagram for a method 34 of using probe 20, in accordance with some embodiments of the present invention.

Method 34 begins at a probe-inserting step 36, at which the probe is inserted into the body of the subject. Following the insertion of the probe, the probe is navigated, at a probe-navigating step 38, to a treatment site within the subject, such as a site within the subject's heart.

In some embodiments, the probe is navigated under ultrasound or fluoroscopic imaging. The balloon and/or another portion of the probe may comprise one or more radiopaque markers, which facilitate navigation of the probe under fluoroscopy.

Alternatively or additionally, the probe may comprise a tracking sensor for tracking the location of the probe during the navigation thereof. For example, another electrode, such as a ring electrode disposed around the circumference of the balloon or around the shaft, may be used to pass electric currents between the probe and receiving patches coupled to the torso of the subject, and the location of the probe may be ascertained from these currents, e.g., as described in US Patent No. 5,983,126, whose disclosure is incorporated herein by reference. As another example, the probe may comprise electromagnetic coils configured to output location-indicating signals in the presence of an external magnetic field, e.g., as described in US Patent Nos. 5,391,199, 5,443,489, and 6,788,967 to Ben-Haim, whose respective disclosures are incorporated herein by reference. As yet another example, both of the above-described tracking techniques may be used, e.g., as described in US Patent No. 8,456,182 to Bar-Tal et al., whose disclosure is incorporated herein by reference.

Alternatively or additionally, surface 26 may be shaped to define an aperture (disposed, for example, at the center of the surface), and the probe may be navigated over a guidewire passing through the shaft, through the balloon (e.g., through a lumen of rod 32), and through the aperture in surface 26.

Subsequently to inserting the probe, at a balloon-inflating step 40, the balloon is inflated. Next, at a pressing step 42, the distal portion of the balloon is pressed against intrabody tissue of the subject, such as cardiac tissue of the subject, at the treatment site, typically such that all the electrodes coupled to the distal portion of the balloon contact the tissue.

Finally, at a treatment step 44, electrical energy is applied to the tissue, using the electrodes. In some embodiments, the electrical energy is applied by passing pulses of direct current (DC) electric current between the electrodes, so as to cause IRE of the tissue. In other embodiments, the electrical energy is applied by passing radiofrequency (RF) electric current between the electrodes, so as to cause ablation of the tissue.

It will be appreciated by persons skilled in the art that the present invention is not limited to what has been particularly shown and described hereinabove. Rather, the scope of embodiments of the present invention includes both combinations and subcombinations of the various features described hereinabove, as well as variations and modifications thereof that are not in the prior art, which would occur to persons skilled in the art upon reading the foregoing description. Documents incorporated by reference in the present patent application are to be considered an integral part of the application except that to the extent any terms are defined in these incorporated documents in a manner that conflicts with the definitions made explicitly or implicitly in the present specification, only the definitions in the present specification should be considered.

### ASPECTS OF THE INVENTION

1. A method for performing irreversible electroporation, the method comprising:
   inserting a shaft into a body of a subject;
   subsequently to inserting the shaft, inflating a balloon coupled to a distal end of the shaft; and
   subsequently to inflating the balloon, using a plurality of electrodes coupled to a distal portion of the balloon that is perpendicular to a longitudinal axis of the distal end of the shaft, applying electrical energy to intrabody tissue of the subject.
2. The method according to aspect 1, wherein the tissue includes cardiac tissue.
3. The method according to aspect 1, wherein applying the electrical energy comprises applying the electrical energy so as to cause irreversible electroporation (IRE) of the tissue.
4. The method according to aspect 1, wherein applying the electrical energy comprises applying the electrical energy while all the electrodes coupled to the distal portion of the balloon contact the tissue.
5. The method according to aspect 1, wherein the distal portion of the balloon is perpendicular to the longitudinal axis of the distal end of the shaft by virtue of an angle between (i) the longitudinal axis of the distal end of the shaft and (ii) a normal line to the distal portion of the balloon at any point on the distal portion of the balloon being less than 30 degrees.
6. The method according to aspect 1, wherein the electrodes are arranged in a grid.
7. The method according to aspect 1, wherein the shaft extends through the balloon and is coupled to the distal portion of the balloon.
8. The method according to aspect 1, wherein at least one support rod is proximally coupled to the shaft and is distally coupled to the distal portion of the balloon.
9. The method according to aspect 1, wherein an area of the distal portion of the balloon is at least 10 mm².
10. The method according to aspect 1, wherein the balloon includes a distal surface comprising the distal portion of the balloon.
11. The method according to aspect 10, wherein the distal surface has a circular perimeter.

## Claims

1. Apparatus for irreversible electroporation, the apparatus comprising:
a shaft, configured for insertion into a body of a subject;
an inflatable balloon coupled to a distal end of the shaft; and
a plurality of electrodes coupled to a distal portion of the balloon that is perpendicular to a longitudinal axis of the distal end of the shaft when the balloon is inflated.

2. The apparatus according to claim 1, wherein the distal portion of the balloon is perpendicular to the longitudinal axis of the distal end of the shaft by virtue of an angle between (i) the longitudinal axis of the distal end of the shaft and (ii) a normal line to the distal portion of the balloon at any point on the distal portion of the balloon being less than 30 degrees.

3. The apparatus according to claim 1, wherein the electrodes are arranged in a grid.

4. The apparatus according to claim 1, wherein the shaft extends through the balloon and is coupled to the distal portion of the balloon.

5. The apparatus according to claim 1, further comprising at least one support rod proximally coupled to the shaft and distally coupled to the distal portion of the balloon.

6. The apparatus according to claim 1, wherein an area of the distal portion of the balloon is at least 10 mm².

7. The apparatus according to claim 1, wherein the balloon comprises a distal surface comprising the distal portion of the balloon.

8. The apparatus according to claim 7, wherein the distal surface has a circular perimeter.

9. The apparatus according to claim 7, further comprising one or more sensors coupled to the distal surface.
